## Europäisches Patentamt

## European Patent Office

## Office européen des brevets

(11) Veröffentlichungsnummer: **0 077 806**

**B1**

# (12) EUROPÄISCHE PATENTSCHRIFT

(45) Veröffentlichungstag der Patentschrift:
06.12.89

(21) Anmeldenummer: 82901497.6

(22) Anmeldetag: 04.05.82

(86) Internationale Anmeldenummer:
PCT/DE 82/00100

(87) Internationale Veröffentlichungsnummer:
WO 82/03781 (11.11.82 Gazette 82/27)

(51) Int. Cl.⁴: **A 61 N 1/368**

(54) **HERZSCHRITTMACHER.**

(30) Priorität: 04.05.81 DE 3118098

(43) Veröffentlichungstag der Anmeldung:
04.05.83 Patentblatt 83/18

(45) Bekanntmachung des Hinweises auf die Patenterteilung:
06.12.89 Patentblatt 89/49

(84) Benannte Vertragsstaaten:
DE FR GB NL

(56) Entgegenhaltungen:
DE-A-2 701 104
FR-A-2 082 703
FR-A-2 435 954
GB-A-2 006 015
GB-A-2 026 870
US-A-3 747 604

(73) Patentinhaber: **BIOTRONIK Mess- und Therapiegeräte GmbH & Co Ingenieurbüro Berlin, Sieversufer 8, D-1000 Berlin 47 (DE)**

(72) Erfinder: **NETTELHORST, Herwig, Frhr., v., Pössnecker Strasse 30, D-1000 Berlin 45 (DE)**
Erfinder: **REXHAUSEN, Hermann, Mellinger Strasse 48, D-3200 Hildesheim (DE)**
Erfinder: **SCHALDACH, Max, Turnstrasse 5, D-8520 Erlangen (DE)**
Erfinder: **SCHWEIGGERT, Eugen, Selbitzer Strasse 81a, D-1000 Berlin 22 (DE)**

(74) Vertreter: **Christiansen, Henning, Dipl.- Ing. Henning Christiansen Patentanwalt Pacelliallee 43/45, D-1000 Berlin 33 (DE)**

EP 0 077 806 B1

**Beschreibung**

Die Erfindung betrifft Herzschrittmacher der im Oberbegriff des Anspruchs 1 angegebenen Art wie aus der GB-A-2 026 870 bekannt ist.

Diese nehmen wohl im Atrium als auch im Ventrikel Signale auf, um aufgrund der Zeiten zu denen in den Herzkammern eigene Aktivitäten des Herzens festgestellt werden, diejenigen Zeiten festzulegen, zu denen Stimulationsimpulse an das Herz abzugeben sind.

Bei den bekannten Schrittmachern wird dabei jeweils das Erscheinen eines Impulses in einer der Kammern benutzt, um aufgrund eines Impulses in der einen Herzkammer die Zeit festzulegen, in der ein Stimulationsimpuls an die andere Herzkammer abgegeben wird, wenn dort nicht vorher (außerhalb der Refraktärzeit) eine Eigenaktivität festgestellt wird.

Nachteilig ist dabei, daß die Festlegung der Stimulationszeit für das Atrium aufgrund der Aktivität im Ventrikel dem physiologischen Herzverhalten widerspricht und aufgrund von Ventrikelaktivitäten, welche zeitlichen Schwankungen unterliegen, Stimulationsimpulse im Atrium erzwungen, werden sich in nicht in Synchronität zum vorliegenden physiologischen Sinusrhythmus befinden.

Der im Anspruch 1 angegebenen Erfindung liegt die Aufgabe zugrunde, einen Herzschrittmacher der vorgenannten Gattung anzugeben, welcher unter Vermeidung dieser Nachteile die weitgehende Aufrechterhaltung des herzeigenen Rhythmus auch dann ermöglicht, wenn Ventrikelaktivitäten vorhanden sind, die nicht in natürlicher Zeitrelation zu den Atriumaktivitäten auftreten. (Extra Systolen)

Besonders vorteilhaft ist, daß das Atrium weitgehend die Führung für die sich bei der Stimulation einstellende Herzrate behält, so daß einer Veränderung der Sinusrate ohne Zeitverzögerung gefolgt wird. Zeitliche Verschiebungen der durch Eigenüberleitung hervorgerufenen Aktionen des Herzens im Ventrikel führen daher nicht zu einer Phasenverschiebung nachfolgender Vorhofstimulationen. Diese Betriebsweise entspricht dem natürlichen Herzverhalten auch deswegen, weil das Herz Veränderungen in der zeitlichen Aufeinanderfolge von P- und U-Impulsen durch sogenannte "kompensatorische Pausen" auszugleichen sucht.

Trotz der damit gegebenen relativen Starrheit der Ankopplung der Schrittmachersteuerung an den Sinusknoten sind die erfindungsgemäßen Schaltmittel in der Lage, diese Kopplung gegebenenfalls aufzuheben und auf die jeweils optimale zeitliche Kopplung überzugehen. Im Zusammenhang mit den im Ausführungsbeispiel dargestellten Mitteln zur Umschaltung der Betriebsarten des Schrittmachers in Abhängigkeit von im Atrium bzw. Ventrikel festgestellten Stör- oder Tachykardiezuständen und Extrasystolen kann bei derart irregulärer Signalaufnahme im Atrium kurz- oder längerfristig die Führung der Stimulation auch dem Ventrikel übertragen werden. Insbesondere bei vorzeitigen Ventrikelkontraktionen wird die Synchronisation nachfolgen Atriumaktivitäten an den Zeitpunkt der Ventrikelaktivität geknüpft, weil insoweit dieses Ereignis auch bei der physiologischen Eigensteuerung des Herzens bedeutungsmäßig überwiegt und somit durch entsprechende Synchronisation des Atriums auch die zeitliche Steuerung des künstlichen Schrittmachers sich an einer nachfolgenden zu erwartenden ebenfalls entsprechend vorzeitig erscheinenden Ventrikelaktivität orientieren muß.

Die Unterdrückung der Synchronisation des die Stimulationszeiten bestimmenden Zeitgebers des Schrittmachers durch im Ventrikel aufgenommene Impulse, welche nach der Bezugszeit erscheinen, die Eigenaktivitäten oder Stimulationszeiten im Atrium zugeordnet ist (und gegebenenfalls nach einer durch die Abgabe eines Stimulationsimpulses im Atrium ausgelöste Austastzeit der Eingangsstufe für im Ventrikel aufgenommene Signale erscheinen), besteht bei einem multi-programmierbaren Schrittmacher dann, wenn eine Betriebsart eingeschaltet ist, welche die Signalaufnahme im Atrium ermöglicht.

Die Refraktärzeitberechnung für die Signalaufnahme aus dem Atrium erfolgt bei nicht erfolgter Synchronisation des Zeitgebers für die Atriumstimulation mit Bezug auf das im Ventrikel aufgenommene Signal, so daß die notwendige Sicherheit in bezug auf Überleitungen und daraus möglicherweise resultierende Stimulationen zu unerwünschten Zeiten gegeben ist. Die Refraktärzeit bildet bevorzugt einen festen Bruchteil des Intervalls, nach dessen Ablauf ein Stimulationsimpuls an das Atrium abgegeben wird.

Die Erfindung basiert auf dem Bestreben, eine universelle Schrittmacherstruktur anzugeben, welche Fehlermöglichkeiten bekannter Systeme vermeidet, wobei die Realisierung in einen implantierbaren System möglich sein soll. Das System soll eine weitestgehende Auswertung der aufgrund von vom Herzen abgeleiteten Signalen zur Verfügung stehender Informationen ermöglichen, wobei der Einfluß von Störsignalen gering gehalten ist. Durch die physiologisch korrekte Zuordnung aller die Betriebsarten des Schrittmachers betreffenden Umschaltvorgänge wird eine große Patientensicherheit erreicht.

Vorteilhafte Weiterbildungen sind in den Unteransprüchen angegeben und werden einschließlich der Erfindung anhand des in den Figuren dargestellten Ausführungsbeispiels näher erläutert. Es zeigen:

Figur 1: ein Blockschaltbild der den erfindungsgemäßen Schrittmacher bildenden Baugruppen unter besonderer Berücksichtigung der im Betrieb auftretenden logischen Verknüpfungen,

Figur 2: ein Zeitdiagramm zur Erläuterung des Funktionsablaufs bei dem in Fig. 1 dargestellten Schrittmacher,

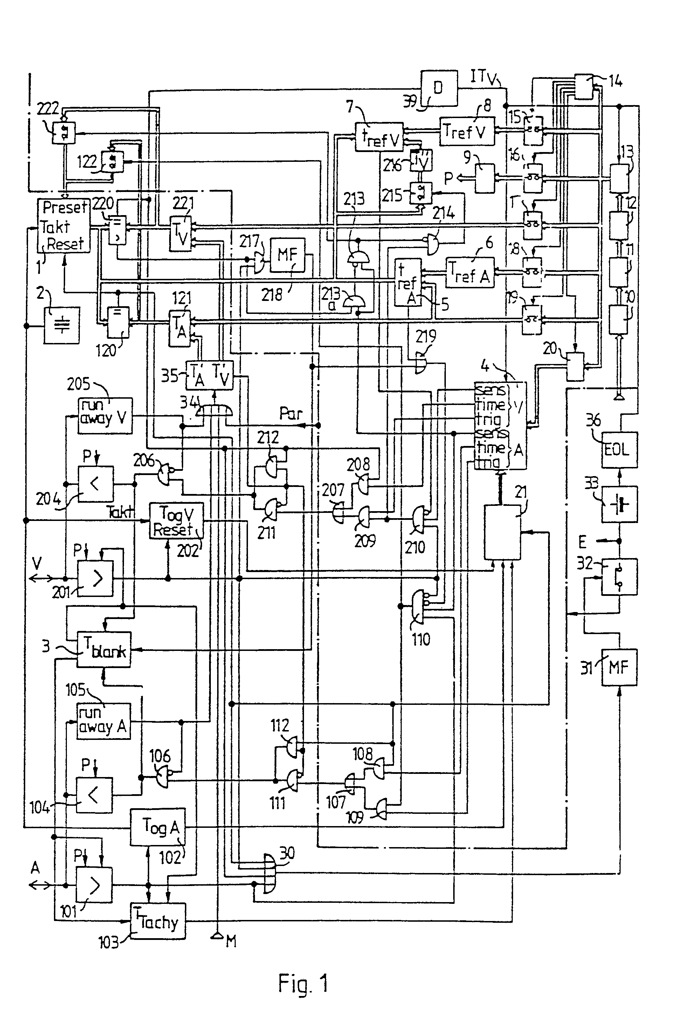

Das in Figur 1 dargestellte in Blockschaltungsform wiedergegebene Prinzipschaltbild des erfindungsgemäßen Herzschrittmachers gibt den Signalfluß zwischen den einzelnen Baugruppen und eine Anzahl von logischen Verknüpfungen wieder. Der verwendeten Schrittmacherstruktur liegt das Konzept eines Schrittmachers zugrunde, das auf grund von Zu- und Abschaltungen von Signalverbindungen in unterschiedlichen Betriebsarten (Modes) benutzt werden kann, wobei die Umschaltung zwischen den einzelnen Betriebsarten (die im Folgenden mit den auf dem Gebiet der Herzschrittmachertechnik eingeführten generischen Codes bezeichnet werden sollen) programmiert und/oder signalabhängig erfolgen kann. In seinen komplexeren Modes paßt der Schrittmacher sich dabei auf physiologische Weise dem natürlichen Herzverhalten an.

Die Realisierung des dargestellten Konzepts kann gleichermaßen mittels diskreter oder programmierbarer Logik-Bauelemente erfolgen, wobei in Falle einer hardwaremäßigen Lösung die erforderlichen Verknüpfungen - wie dargestellt - mittels Logik-Gattern und Speichermitteln wie Flip-Flops und Latches erfolgen, während beispielsweise bei einer Mikroprozessorlösung die Logik-Entscheidungen nicht parallel, sondern nach Programm seriell vorgenommen werden.

RAM-Speicherplätze übernehmen dabei die Funktion der Speichermittel zum Festhalten von für Betriebszustände charakteristischen Signalen. Einige der dargestellten Funktionsbaugruppen stellen vorteilhafte Weiterbildungen der Erfindung dar.

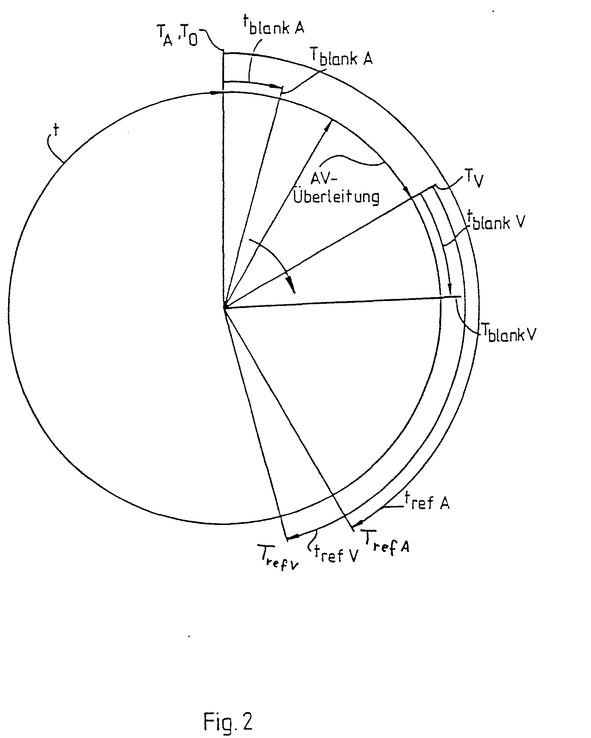

Aus Figur 2 ist der zeitliche Ablauf der Signalverarbeitung und Stimulationsvorgänge ersichtlich, wobei in den übrigen Darstellungen entsprechende Zeitbezeichnungen verwendet wurden, so daß diesbezüglich auf diese Figur stets Bezug genommen werden kann. In der Darstellung fällt der Anfangszeitpunkt $T_0$ zusammen mit dem Zeitpunkt $T_A$, der nach einem Umlauf (entsprechend einem Herzzyklus) erreicht wird und die Aktivität des Atriums charakterisiert. Im Falle einer Stimulation im Ventrikel oder Atrium folgt eine Austastzeit $t_{blankA}$ für die im Atrium plazierte Elektrode, wobei die Dauer dieser Austastzeit jeweils davon abhängig ist, ob im Atrium oder im Ventrikel, d.h. in derselben oder in der jeweils anderen Kammer stimuliert wurde. Das Ende dieser Austastzeit ist mit $T_{blankA}$ bezeichnet.

Zwischen $T_A$ und $T_V$ befindet sich die AV-Überleitungszeit, wobei $T_V$ den Zeitpunkt der Aktivität des Ventrikels charakterisiert. An $T_V$ schließt sich entsprechend eine Austastzeit $t_{blankV}$ an, welche im Zeitpunkt $T_{blankV}$ endet. Die Refraktärzeit des Atriums $t_{REFA}$ beginnt mit $T_0$ und endet bei $T_{REFA}$, während die Refraktärzeit des Ventrikels bei $T_V$ beginnt und bei $T_{refV}$ endet. Die Zeiten $T_{og}$ und $T_{tachy}$ sind aus dem Diagramm gemäß Figur 2 nicht direkt ersichtlich, da diese durch entsprechende Folgeraten der Herzaktionspotentiale und/oder Störsignale als Triggersignale festgelegt werden.

Bei dem in Figur 1 dargestellten Herzschrittmacher bilden die Anschlüsse A und V Ein- und Ausgänge für Signale, welche vom Herzen aufgenommen werden bzw. in Form von Stimulationsimpulsen an das Herz abgegeben werden. Mit "A" ist dabei der Anschluß für eine im Atrium und mit "V" der Anschluß für eine im Ventrikel fixierte Elektrode bezeichnet.

Die zentrale Steuerung für die zeitliche Zuordnung der abzugebenden Impulse erfolgt über einen Zähler 1, welcher durch von einem Taktgenerator 2 erzeugte Impulse inkrementiert wird und über einen "Reset"-Eingang in eine Ausgangsstellung zurücksetzbar ist. In Abhängigkeit von vom Herzen aufgenommenen Signalen ist dieser Zähler über einen zusätzlichen "Preset"-Eingang auf beliebige Zählerstände in Vorwärtsrichtung - entsprechend einer zeitlichen Voreilung - voreinstellbar. Ein Verändern des Zählerstandes in Rückwärtsrichtung ist nicht notwendig. Nach Erreichen seines Endstandes setzt der nachfolgende Impuls den Zählen auf 0, von wo aus der Zählvorgang fortgesetzt wird. Das Zusammenwirken des Zählers mit den übrigen in Figur 1 dargestellten Bauelementen wird weiter unten näher beschrieben. (Die Übertragung einzelner digitaler Signalimpulse oder analoger Signale ist in den Figuren durch einfache Verbindungen dargestellt, während doppelte Linien die Übertragung vollständiger Datenwörter symbolisieren.)

Die vom Atrium über eine dort fixierte Elektrode aufgenommenen Signale gelangen über den Anschluß A an eine Eingangsstufe 101, welche einen Vorverstärker enthält, der über einen Programmiereingang (zugehöriger Pfeil P) in seinem Verstärkungsfaktor einstellbar ist. Die Eingangsstufe bewirkt gleichzeitig eine Impulsformung in der Weise, daß die vom Atrium aufgenommenen Signale in Form von impulsförmigen Logiksignalen in den nachfolgenden Schaltstufen weiterverarbeitet werden können. Die Darstellung der Logikpegel bei dem wiedergegebenen Ausführungsbeispiel erfolgt in positiver Logik, d.h. das Vorhandensein eines Signals wird mit dem H-Zustand und die Abwesenheit mit dem L-Zustand bezeichnet. Umschaltelemente (Zähler, Mono- und Flip-Flops) werden durch die ansteigenden Vorderflanken der Signalimpulse aktiviert. Weiterhin weist die Eingangsstufe 101 Filtermittel auf, mit einer Filtercharakteristik, die sie vorzugsweise für die aufzunehmenden Nutzsignale durchlässig macht.

Die Eingangsstufe 101 wird außerdem von einer Austastschaltung 3 beeinflußt, welche die Eingangsstufe für vorgegebene Zeiträume $t_{blankA}$ sperrt, wenn ein Stimulationsimpuls an Atrium oder Ventrikel abgegeben wurde. Auf diese Weise wird verhindert, daß ein vom Schrittmacher abgegebener Stimulationsimpuls als herzeigenens Signal verarbeitet wird und das Betriebsverhalten des Schrittmachers fehlerhaft beeinflußt.

Der Eingangsstufe nachgeschaltet ist eine Störerkennungsschaltung 102, welche ein Ausgangssignal abgibt, wenn der Abstand zweier aufeinanderfolgender, von der Eingangsstufe 101 verarbeiteter Signalimpulse einen vorgegebenen zeitlichen Mindestabstand ($T_{og}$), entsprechend einer maximalen Folgefrequenz, unterschreitet.

Derartige eine Störung bildende Signale können ihre Ursache sowohl außerhalb (elektromagnetische Einstreuungen oder Wechselströme) als auch innerhalb des Körpers des Patienten (Elektrodenartefakte) haben. Die Störerkennungsschaltung 102 verhindert im Zusammenwirken mit weiter unten zu beschreibenden Mitteln, daß an der Atriumelektrode aufgenommene Impulse, welche die vorgegebene Frequenz überschreiten, zur Weiterverarbeitung zugelassen werden.

Der Ausgang der Eingangsstufe 101 ist weiterhin mit dem Eingang einer Tachykardieerkennungsstufe 103 verbunden, welche entsprechend ein Signal abgibt, wenn die am Ausgang der Stufe 101 erscheinenden Impulse eine vorgegebene Folgezeit unterschreiten. Die "Tachykardierate" $T_{Tachy}$ ist mit ca. 250 ms größer als die als Kriterium für das Vorhandensein eines Störsignals festgelegte Zeitdauer $T_{og}$, welche ca. 100 ms beträgt.

Der Anschluß V, an den eine im Ventrikel zu fixierende Elektrode angeschlossen werden kann, ist mit dem Eingang einer weiteren Eingangsstufe 201 verbunden, die in ihren grundsätzlichen Funktionen der Eingangsstufe 101 für vom Atrium aufgenommene Signale entspricht. Der Verstärkungsfaktor ist über einen zusätzlichen Eingang (zugehöriger Pfeil P) einstellbar. Eine separate Störerkennungsschaltung 202 für die von der im Ventrikel fixierten Elektrode aufgenommenen Störsignale gibt ein Ausgangssignal ab, wenn die vom Ventrikel aufgenommenen Signalimpulse eine höhere als eine vorgegebene Frequenz aufweisen. Die Eingangsstufe 201 wird entsprechend durch von der Austaststufe 3 abgegebene Signale zeitweise gesperrt.

Stimulationsimpulse für das Atrium werden mittels einer Impulsformerschaltung 104 erzeugt, wobei die Amplitude der Stimulationsimpulse über einen zusätzlichen Eingang (zugehöriger Pfeil P) einstellbar ist. Die Impulsformerstufe 104 wird durch kurzzeitige Impulse mit logischem H-Pegel angesteuert und enthält auch die zur Heraufsetzung der Eingangsimpulse auf den zur Stimulation notwendigen Pegel erforderlichen Verstärkungsmittel.

Die von der Impulsformerstufe 104 abgegebenen Impulse gelangen einerseits zum Anschluß A und andererseits zu einer Run-away-Schutzschaltung 105, welche in dem dargestellten Ausführungsbeispiel durch ein Monoflop gebildet wird, dessen Impulsdauer auf die höchste zulässige Stimulationsrate im Atrium abgestimmt ist. Auf einen Ausgangsimpuls von der Impulsformerstufe 104 hin gibt die Schutzschaltung 105 einen Impuls vorgegebener Breite an den invertierenden Eingang eines UND-Gatters 106 ab, welches daraufhin für die Dauer des letztgenannten Impulses hin für seinen anderen Eingang gelangende Signalimpulse gesperrt ist.

Eine Impulsformerstufe 204 für zum Stimulieren des Ventrikels bestimmte Impulse ist ebenfalls über einen weiteren Eingang (zugehöriger Pfeil P) bezüglich der Amplitude der abgegebenen Impulse beeinflußbar. Eine separate Run-

away-Schutzschaltung 205 für den Ventrikel sperrt ein UND-Gatter 206 über dessen invertierende Eingang, wenn die Folgezeit der den Ventrikel stimulierenden Impulse den vorgegebenen Wert unterschreitet.

Die Zeiten, zu denen Stimulationsimpulse abgegeben werden können (mit $T_A$ und $T_V$ bezeichnet), bestimmt der Zähler 1, wobei jeweils für Atrium und Ventrikel separate Vergleicherschaltungen 120 bzw. 220 vorgesehen sind, welche einen Impuls am Ausgang erzeugen, wenn der Zählerstand des Zählers 1 mit einem vorgegebenen Zählerstand in einem Speicher 121 für den Stimulationszeitpunkt des Atriums bzw. einem entsprechenden Speicher 221 für den Ventrikel übereinstimmt. Die Vergleicherschaltung 220 gibt über einen zusätzlichen Ausgang "" ein Signal ab, wenn der Zählerstand des Zählers größer ist als der in dem Speicher 221 festgehaltene Wert für $T_V$. Die genannten Zeitmarken $T_A$ und $T_V$ bilden Bezugszeiten für den Betrieb des Schrittmachers und beenden im Demand-Betrieb die sogenannten "Escape-Intervalle" innerhalb denen eine entsprechende Eigenaktion des Herzens die Erzeugung eines Stimulationsimpulses durch den Schrittmacher verhindert.

Die Zeiten $T_A$ und $T_V$ für Atrium und Ventrikel in den Speichern 121 bzw. 221 werden in Form von Zahlenwerten programmiert, welche diejenigen Zählerstände repräsentieren, die der Zähler 1 im Stimulationszeitpunkt erreicht haben muß. Die Zeitzuordnung zu den Zählerständen erfolgt in der Weise, daß die Stimulationszeitpunkte mit einem ausreichend feinen Zeitraster einstellbar sind. Bei einem Zählertakt von beispielsweise 1 kHz weist das zur Verfügung stehende Zeitraster eine Teilung in Millisekunden auf. Die mittels der Speicher 121 bzw. 221 vorgebbaren Zählerstände sind durch weiter unten darzustellende Mittel veränderbar.

Die Eingangsimpulse für die Ausgangsstufe 104, welche das UND-Gatter 106 passieren, stammen vom Ausgang eines ODER-Gatters 107, an dessen Eingänge alternativ die über die Abgabe eines Stimulationsimpulses an das Atrium entscheidenden Signale gelangen. Einer der Eingänge des ODER-Gatters 107 ist mit dem Ausgang eines UND-Gatters 108 verbunden, das ein Ausgangssignal abgibt, wenn die Vergleicherschaltung 120 das Erreichen des Zeitpunktes $T_A$ angibt und damit anzeigt, daß im Demand-Zustand der Vorhofstimulation ein Zeitpunkt erreicht wurde, bei dem wegen fehlender eigener Aktivität des Herzens ein Stimulationsimpuls erforderlich ist. Das UND-Gatter 108 wird über seinen weiteren Eingang mittels eines Signals durchgeschaltet, welches von einem Ausgang "time$_A$" des Betriebszustandsregister 4 abgegeben wird, wenn eine Impulsabgabe nach Zeitablauf erwünscht ist. Bei freigegebener Signalaufnahme (sens$_V$ bei Block 4) für die entsprechende Kammer erfolgt damit ein "Demand"-Betrieb mit Inhibierung durch herzeigene Impulse, während bei gesperrter Signalaufnahme eine Stimulation zu festen Zeiten erfolgt.

Ein weiteres UND-Gatter 109 wird über einen entsprechenden Ausgang "trig$_A$" der Schaltung 4 bei durch Herzeigenaktionen im Vorhof getriggerter Signalabgabe an den Vorhof aktiviert. Das Signal zum synchronen Auslösen des Stimulationsimpulses im Vorhof gelangt an den Eingang des UND-Gatters 109 vom Ausgang eines weiteren UND-Gatters 110, das seinerseits durch ein von einem Ausgang "sens$_A$" der Schaltung 4 an seinen Eingang gelangenden Signal aktiviert wird, wenn die Aufnahme von Signalen aus dem Vorhof bei der gewählten Betriebsweise des Schrittmachers vorgesehen ist.

Es ist ersichtlich, daß für den durch herzeigene Signale getriggerten Betrieb diese Freigabe der Signalaufnahme Voraussetzung ist. An das UND-Gatter 110 gelangt nicht nur das Ausgangssignal der Eingangsstufe 101, sondern an dessen weiteren invertierenden Eingang auch das Ausgangssignal einer Refraktärstufe für den Vorhof 5, welche ein Signal abgibt, wenn für einen Zeitraum $t_{refA}$, der mit dem Erreichen des den Zeitpunkt $T_A$ entsprechenden Zählerstand durch den Zähler 1 beginnt, die Verarbeitung der im Atrium aufgenommenen Signale zur Auswertung für die Abgabe von Stimulationsimpulsen gesperrt ist. Ein der Refraktärzeit $T_{refA}$ für das Atrium entsprechender Zahlenwert ist in einem Speicher 6 festgehalten und über weiter unten dargestellte Programmiermittel durch äußere Signale veränder-_ bar. Ein vom Ausgang der Schaltung 201 im Falle der Signalaufnahme im Ventrikel gelangendes Signal sperrt über dessen weiteren invertierenden Eingang das UND-Gatter 110, so daß bei gleichzeitig im Vorhof und Ventrikel ankommenden Signalen, das Vorhofsignal gesperrt ist und somit der Ventrikel Priorität hat.

Ein ODER-Gatter 207 und UND-Gatter 208 bis 210 bilden die entsprechenden, über die Abgabe von Stimulationsimpulsen an den Ventrikel bzw. über die Signalaufnahme aus dem Ventrikel entscheidenden Logik-Gatter. Das gewünschte Betriebsverhalten des Schrittmachers läßt sich ebenfalls über drei Ausgänge "sens$_V$", "time$_V$" und "trig$_V$" der Schaltung 4 bestimmen, wobei das an den weiteren Eingang des UND-Gatters 208 gelangende Signal die Stimulation freigibt, wenn der Stand des Zählers 1 den im Speicher 221 festgehaltenen, den Zeitpunkt $T_V$ zugeordneten Zählerstand erreicht und somit zu diesem Zeitpunkt ein Ausgangssignal des Vergleichers 220 an das UND-Gatter 208 ausgegeben wird.

Die synchrone Stimulation wird durch das UND-Gatter 209 ausgelöst, dem das Ausgangssignal des UND-Gatters 210, welches die vom Ventrikel aufgenommenen Signalimpulse weiterleitet, zugeführt wird. Das UND-Gatter 210 ist über seinen invertierenden Eingang während der Ventrikel-Refraktärzeit $t_{refV}$ gesperrt, welche mittels eines Vergleichers 7 festgelegt wird, wobei der erreichte Zählwert des Zählers 1 mit dem in dem Speicher 8 festgehaltenen, die Zeitdauer $T_{refV}$ repräsentierenden Zählwert verglichen wird und zu dem Inhalt des Speichers 8 derjenige des Speichers 216, dessen Inhalt $T_V$ im Normalfall

der Zeit $T_V$ entspricht, hinzuaddiert wird. (Unterschiede zwischen der Funktion der Speicher 6 für die Refraktärzeit des Atriums und 7 für die Refraktärzeit des Ventrikels sind aus der folgenden Beschreibung ersichtlich.) Der Inhalt des Speichers 8 ist wie derjenige des Speichers 6 über äußere Programmiermittel veränderbar.

Die Ausgangssignale der UND-Gatter 110 bzw. 210 beeinflussen des weiteren Schalter 122 und 222, mittels denen der Zähler 1 über seinen Preset-Eingang auf die in den Speichern 121 (für den Wert $T_A$) bzw. 221 (für den Wert $T_V$) vorhandenen werte voreinstellbar ist. Damit wird der Zähler 1 beim Erscheinen eines intrakardialen Signals im Atrium auf den der Zeit $T_A$ bzw. beim Erscheinen eines Signals im Ventrikel auf den der Zeit $T_V$ entsprechenden Zahlenwert vorangesetzt. Auf diese Weise wird der Schrittmacher mit dem Herzverhalten synchronisiert, wenn die UND-Gatter 110 bzw. 210 über ihre entsprechenden Signaleingänge für die Signalaufnahme vom Herzen freigegeben sind. Sind die zuletzt genannten Gatter gesperrt, so e(folgt die Stimulation gegebenenfalls asynchron.

Die Inhalte der Speicher 4, 6 und 8 sowie der eines weiteren Speichers 9, welcher die programmierbaren Verstärkungsfaktoren bzw. Ausgangssignalamplituden repräsentierenden Daten für die Stufen 101 und 201 sowie 104 und 204 (zugeordnete Pfeile P) enthält, sind über die blockschaltungsmäßig dargestellten Programmiermittel zu beeinflussen, welche nachfolgend kurz beschrieben werden sollen:

Die von außerhalb des Schrittmachers über einen geeigneten Nachrichtensignalträger (radiofrequente, optische oder sonstige Signale) in Form von Impulsen aufmodulierten, von einem entsprechenden - nicht dargestellten - Sender abgegebenen Signale, gelangen zu einem Empfänger 10, der die verstärkten Signale einem Dekoder 11 zuleitet, mit dessen Hilfe die zu übertragene ursprüngliche Programmierimpulsfolge wieder hergestellt wird. In einer Prüfstufe 12 wird mittels zusätzlich übertragener redundanter Signale festgestellt, ob die übertragene Information vollständig ist und letztere zutreffendenfalls an einen Steuerbaustein 13 übermittelt, durch den mittels einer ersten Teilinformation eine Auswahlschaltung 14 entsprechend aktiviert wird.

Durch das Ausgangssignal der Schaltung 14 wird eine der Schaltstufen 15 bis 20 betätigt, so daß die zweite übertragene Teilinformation in den mit der aktivierten Stufe der Stufen 15 bis 20 verbundenen Speicher gelangt und dort festgehalten wird. Die bereits erwähnten Speicherstufen 4, 6, 8, 9 sowie 121 und 222 halten die für den Betrieb des Schrittmachers wesentlichen veränderbaren Daten fest, wie sie in der vorangehenden Beschreibung aufgeführt wurden.

Der Speicher 4 ist von zusätzlichen Signalen beeinflußbar, welche aus der Signalverarbeitungsstufe 21 stammen, und eine Umschaltung der Betriebszustände in Abhängigkeit von vom Schrittmacher selbst aus den Herzen aufgenommenen Signalen bewirkt, wobei die Eingangssignale der Stufe 21 von den Stufen 102 und 202 stammen und im Atrium bzw. im Ventrikel aufgenommene Störsignale repräsentieren, sowie von der Tachykardiestufe 103, welche ein Ausgangssignal abgibt, wenn der Tachykardiezustand festgestellt wurde.

Die von der strichpunktierten Linie umgebenen Bauelemente zur Signalverarbeitung und -speicherung sind jeweils nur zeitweise aktivert, da die zum Betrieb des Schrittmachers notwendigen logischen Operationen mittels moderner mikroelektronischer Bauelemente in sehr kurzen Zeiten ausgeführt werden können, so daß deren permanenter Betrieb einen unnötigen Energieverbrauch zur Folge haben würde. Die außerhalb der genannten Linie befindlichen Bauelemente, die entweder direkt mit den im Herzen fixierten Elektroden verbunden sind und damit die Signalaufnahme vom Herzen kontrollieren, bzw. diejenigen Mittel, welche die Zeitgeber- und Zeitvergleichermittel bilden, sind dauernd aktiviert, da durch letztere bestimmt wird, zu welchen Zeitpunkten logische Operationen auszuführen sind.

Die Aktivierung der innerhalb der strichpunktierten Linie befindlichen Bauelemente erfolgt durch die von den Vergleichermitteln 120 bzw. 220 abgegebenen Signale, wenn ein vorgegebener Zeitpunkt erreicht wurde, oder aber durch die Ausgangssignale der Eingangsstufen 101 bzw. 201, wenn nämlich vom Herzen (außerhalb der jeweiligen Refraktärzeit) ein Signal aufgenommen wurde. Die Zusammenfassung der genannten Signale erfolgt über ein ODER-Gatter 30, welches über ein Monoflop 31 einen Schalter 32 betätigt, der die in der gewählten Darstellung innerhalb der strichpunktierten Linie angeordneten Bauelemente mit einer Energiequelle 33 verbindet, wobei dieses Verbinden mit einer Energiequelle in jedem Heraufsetzen der Energieversorgung, also auch in einem Umschalten der betreffenden Bauelemente von einem Bereitschafts-(Stand-by-) Zustand in einen Betriebszustand bestehen kann.

Die Impulsdauer des Monoflops 31 ist dabei so bemessen, daß zum Ausführen der notwendigen Schaltvorgänge ein ausreichend langer Zeitraum zur Verfügung steht. Diskrete Logikbauelemente lassen sich energiesparend bei derart herabgesetzter Versorgungsspannung betreiben, daß zwar keine Veränderungen vorgenommen werden können, gespeicherte Signalzustände jedoch erhalten bleiben. Diese Bauelemente dienen der nachfolgenden Signalverarbeitung in dem Sinne, daß sie festlegen, ob auf ein Eingangssignal oder beim Erreichen eines vorgegebenen Zeitpunkts ein Stimulationsimpuls abgegeben werden soll.

Der von der Energiequelle 33, die im dargestellten Ausführungsbeispiel von einer Batterie gebildet wird, ausgehende Pfeil E deutet an, daß die außerhalb der strichpunktierten Linie angeordneten Bauelemente direkt von der Energiequelle 33 ohne Zwischenschaltung des Schaltelementes 32 versorgt werden.

Mit der dargestellten Anordnung sind die Betriebsfunktionen üblicher Schrittmachertypen mit synchroner oder asynchroner Stimulation im Atrium und/oder Ventrikel erzeugbar, wobei je nach Zustand der Schaltmittel 4 und Freigabe der entsprechenden Stimulationssignale bzw. Signalaufnahmemöglichkeit über die UND-Gatter 108 bis 110 bzw. 208 bis 210 auch jene Schrittmacherbetriebsarten höherer Ordnung möglich sind, wie sie in Form einer Übersicht beispielsweise in dem Aufsatz "Physiological Cardiac Pacing", R. Sutton, J. Perrins und P. Citron in "PACE"; Vol. 3, März-April 1980, S. 207 bis 219 wiedergegeben sind.

Das grundsätzliche Stimulationsverhalten der verschiedenen Schrittmacherbetriebsweisen bei verschiedenartigen Herzsignalen ist ebenfalls in der vorgenannten Literaturstelle angegeben.

Weitere Einzelheiten zu den in der nachfolgenden Beschreibung dargestellten Schaltungsteilen können den am gleichen Tage eingereichten, diese Schaltungsteile wegen der damit verbundenen Neuerungen näher beschreibenden Patentanmeldungen WO 82/3780 und WO 82/3782 bis WO 82/3786 derselben Anmelderin entnommen werden.

Durch die funktionsmäßige Trennung des komplexen, nur zeitweise aktivierten Logiksystems von den permanent betriebsbereiten Bauelenenten, ist es in vorteilhafter Weise möglich, für den Fall des Ausfalls des umfangreichen Logikteils ein einfaches Ersatzsystem vorzusehen, durch welches ein Notbetrieb aufrecht erhalten werden kann. Obgleich zwar einerseits die Sicherheit einzelner Bauelemente gegen Ausfall in der Vergangenheit wesentlich gesteigert werden konnte, wird andererseits angestrebt, die Informationsverarbeitung in einem künstlichen Herzschrittmacher möglichst allen therapeutischen Anforderungen gerecht werden zu lassen, um dem Patienten eine optimale Stimulationstherapie zukommen zu lassen, und damit die erreichte Erhöhung der Zuverlässigkeit zum Teil wieder kompensiert.

Das dargestellte Konzept gestattet es, die Vorteile eines einfachen, höchst betriebssicheren Systems mit einem solchen höherer Verarbeitungsfähigkeit derart zu verbinden, daß bei Ausfall des komplexen Datenverarbeitungssystems ein einfaches Ersatzsystem bereitsteht, welches in der Lage ist, die Grundfunktionen des Schrittmachers unter nahezu allen Umständen für einen nur durch die Betriebsdauer der Energiequellen begrenzten Zeitraum sicherzustellen.

Diese Ersatzschaltung kann prinzipiell durch verschiedenartige Signale aktiviert werden. Eines dieser Signale ist ein von der komplexen Logikschaltung ausgehendes Signal "Par", das bei einer mittels eines Mikroprozessors realisierten Logikschaltung dann erscheint, wenn innerhalb der durchgeführten Operationen ein Plausibilitätsfehler ermittelt wurde, wie er beispielsweise durch eine Paritätsüberprüfung mit Hilfe von redundanten Signalen in bekannter Weise erzeugt werden kann. Entsprechende Signale lassen sich auch aus mit diskreten Logikbauelementen arbeitenden Schaltungen gewinnen, wenn hier zusätzliche Elemente verwendet werden, welche unzulässige Betriebszustände, beispielsweise in Form von Signalen, erkennen, die bei korrekt arbeitenden Logikmitteln beispielsweise nicht gleichzeitig oder aber nicht mit einer oberhalb oder unterhalb einer vorgegebenen Grenzfrequenz liegenden Rate etc. erscheinen dürfen. Derartige ein Fehlverhalten aufzeigenden Signale werden auch von den Ausgängen der Run-away-Schutzschaltungen 105 und 205 gewonnen und über ein ODER-Gatter 34 zusammengefaßt. Bei der dargestellten Anordnung kann es theoretisch in dem Fall zu einen Ansprechen der Run-away-Schutzschaltungen bei ordnungsgemäß arbeitenden Taktgenerator 2 kommen, wenn in die Speicher 121 oder 221 durch einen Fehler in dem durch die strichpunktierte Linie umrandeten Teil der Logikschaltungen fehlerhafte Zahlenwerte eingelesen wurden.

Dem ODER-Gatter 34 wird ein weiteres Signal M zugeführt, welches von einem Reed-Schalter stammt, der in der Zeichnung nicht dargestellt ist. Auf diese Weise ist es möglich, mittels eines von außen einwirkenden Magnetfeldes den Ersatzbetriebszustand entsprechend der "Magnetfrequenz" der bekannten Schrittmacher einzustellen.

Der Ausgang des ODER-Gatters 34 aktiviert eine Speicherschaltung 35, welche Zeitpunkten $T_A'$ und $T_V'$ entsprechende Zahlenwerte enthält, die bei Aktivierung dieser Speicherschaltung 35 mit Vorrang in die Speicher 121 bzw. 221 eingelesen werden. Gleichzeitig wird von der Schaltung 35 ein Ausgangssignal abgegeben, welches über invertierende Eingänge von UND-Gattern 111 und 211, die zwischen die Gatter 107 und 106 bzw. 207 und 206 eingeschaltet sind und diesen Signalweg sperren, während über die somit aktivierten UND-Gatter 112 und 212 eine direkte Verbindung zu den Ausgängen der Vergleicher 120 und 220 hergestellt wird.

Damit ist das im "DOO-Betrieb arbeitende Ersatzsystem nicht mehr von der Signalverarbeitung der innerhalb der strichpunktierten Linie befindlichen Elemente der Schaltung abhängig. Die Werte $T_A'$ und $T_V'$ werden dabei so gewählt, daß sich eine für die Frequenzen annehmbare Ersatz-Stimulationsfrequenz ergibt. Die Betriebsart "DOO" bezieht sich auf den Anschluß zweier Elektroden. Ist jeweils nur eine der beiden Elektroden angeschlossen, so arbeitet der Schrittmacher im Zustand "AOO" oder "VOO" und gibt damit entsprechend der gewählten Elektrodenkonfiguration ebenfalls die zur Aufrechterhaltung der vitalen Funktionen notwendigen Stimulationsimpulse ab.

Mittels zweier in die Aktivierungsleitung des Schalters 222 eingefügter UND-Gatter 213 und 213a wird eine Synchronisation des Zählers 1 (und damit der Zeitsteuerung des Schrittmachers) auf vom Ventrikel erscheinende Signale für die Zeit der Überleitung vom Atrium zum Ventrikel verhindert (AV-Überleitung zwischen $T_A$ und $T_V$ in Figur 2), wenn gleichzeitig eine Signalaufnahme im Atrium möglich ist. Die entsprechenden Zeitmarken beziehen sich (abhängig von der Betriebsart des Schrittmachers) auf Zeiträume zwischen herzeigenen oder stimulierten Signalen. Dem UND-Gatter 213a werden das Signal sensA des Speichers 4 und das Signal ''' des Vergleichers 220 an seinen Eingängen zugeführt. Das Ausgangssignal des UND-Gatters 213a sperrt gegebenenfalls das UND-Gatter 213 über dessen invertierenden Eingang. Der weitere Eingang des UND-Gatters ist mit den die Signalaufnahme für das Atrium am UND-Gatter 110 freigebenden Signalausgang des Speichers 4 verbunden. Damit wird erreicht, daß eine Synchronisation bei regulärer Ventrikeltätigkeit stets nur mit $T_A$ erfolgt und somit das Atrium bei AV-sequentieller Stimulation die Führung behält bzw. bei fehlender Signalaufnahme aus dem Atrium der Abstand aufeinanderfolgender Zeitpunkte $T_A$ konstant bleibt.

Der hier beschriebene Schrittmacher stellt damit die physiologisch richtige Synchronisation mit dem Atrium sicher, wenn dort ungestörte Signale zur Verfügung stehen. Ist die Signalaufnahme im Atrium gestört so e(folgt - entsprechend den vorgesehenen Möglichkeiten der Betriebszustandsänderungen, die weiter unten beschrieben werden - eine Umschaltung in einen eine Signalaufnahme im Ventrikel ermöglichenden Betriebszustand, so daß auch in diesem Fall eine korrekte richtige Stimulation gewährleistet ist.

Obgleich bei freigegebener Signalausnahme im Atrium für die Zeit t zwischen $T_A$ und $T_V$ keine Synchronisation des Zählers 1 durch im Ventrikel aufgenommene Signale erfolgt, wird die Refraktärzeit des Ventrikels dadurch in physiologisch sinnvoller Weise gesetzt, daß diese sich trotz fehlender Synchronisation des Zählers 1 auf die Zeiten bezieht, zu denen die Herzaktion in Ventrikel festgestellt wurde, was bei dem dargestellten Ausführungsbeispiel dadurch realisiert ist, daß das Ausgangssignal des UND-Gatters 210 über ein UND-Gatter 214 und ein Schaltelement 215 einen Speicher 216 mit einem zu diesem Zeitpunkt im Zähler 1 enthaltenen Zahlenwert lädt, so daß dieser Zeitpunkt $T_V''$ als Bezugszeitpunkt jetzt für die Ermittlung der Refraktärzeit $T_{refv}$ durch den Vergleicher 7 dienen kann. Erfolgt eine Signalaufnahme ausschließlich im Ventrikel, so ist eine Synchronisation des Zählers durch das Schaltelement 222 im Zeitpunkt $T_V$ freigegeben, wodurch mittels des UND-Gatters 214 in den Speicher 216 dann derjenige Zahlenwert für $T_V$ übertragen wird, welcher auch im Speicher 221 vorhanden ist.

Nach Ablauf der Refraktärzeit für aus dem Ventrikel aufgenommene Signale erscheinende Impulse, welche von Extrasystolen herrühren, synchronisieren den Zeitzähler 1 des Schrittmachers, was bei dem dargestellten Ausführungsbeispiel dadurch erreicht wird, daß der Zähler 1 durch die Schaltermittel 222 auf den Wert $T_V$, im Speicher 221 vorhanden ist, gesetzt wird. Darüberhinaus muß auch sichergestellt sein, daß retrograd übergeleitete Erregungen nach Extrasystolen keine Synchronisation des Schrittmachers bewirken. Dies wird dadurch erreicht, daß nach Erkennen der Extrasystole (Ventrikelaktivität vor

dem Zeitpunkt T$_O$) die Atriumrefraktärzeit t$_{refA}$ für einen Zyklus auf 400 ms gesetzt wird, so daß für einen ausreichend langen Zeitraum (entsprechend der retrograden Überleitungszeit) - ausgehend von der Zeitmarke T$_V$ das Atrium für eine Signalaufnahme gesperrt ist.

Die Sperrung derartiger retrograder Überleitungen ist deshalb von besonderer Bedeutung, weil ein vom Schrittmacher auf das Signal im Atrium hin mit der vorgegebenen AV-Überleitungszeit abgegebener Stimulationsimpuls im Ventrikel eine Stimulation in den vulnerablen Phase zur Folge hätte.

Um hier den notwendigen Schutz zu bewirken, werden unmittelbar auf eine Extrasystole hin die Signalaufnahmemittel für Vorhof und Ventrikel für einen vorgegebenen Zeitraum refraktär geschaltet. Und zwar wird bei einem Eingangsimpuls am UND-Gatter 217 vom Ausgang der Eingangsstufe 201 für den Ventrikel, der erscheint, wenn auch der Vergleicher 220 am Ausgang "" ein Signal abgibt, das an den zweiten Eingang des UND-Gatters 217 gelangt, ein Monoflop 218 gestartet. Dieses Monoflop gibt daraufhin einen Ausgangsimpuls von ca. 400 ms Dauer ab, welcher an einem Eingang des ODER-Gatters 219 gelangt, dessen zweiter Eingang mit dem Ausgang des Vergleichers 5 für die Refraktärzeit T$_{refA}$ verbunden ist, und dessen Ausgangssignal zu einem der Eingänge des UND-Gatters 110 übertragen wird.

Auf diese Weise ist sichergestellt, daß bei jeder nach der Zeit T$_V$ festgestellten Kammerkontraktion die Refraktärzeit für das Atrium neu gestartet und für die Zeitdauer des von dem Monoflop 218 abgegebenen Impulses (vorzugsweise 400 ms) aufrechterhalten wird, so daß eine Synchronisation des Schrittmachers durch retrograde Überleitungen verhindert ist. (Wie weiter unten näher erläutert ist, sind für diesen Zeitraum auch die Stör- und Tachykardieerkennungsmittel gesperrt, da diese bei einer retrograden Überleitung nicht ansprechen sollten. Die erforderliche Signalverbindung wird durch die Leitung vom Ausgang des Monoflops 218 zum Block 3 gebildet.) Ein ODER-Gatter sperrt auch den Ventrikel, wobei bevorzugt mittels - nicht dargestellter Schaltmittel - statt der Impulszeit des Monoflops 218 auch die in Block 7 enthaltene Zeit heranziehbar ist.

Für den Fall, daß gleichzeitig Signale im Atrium und Ventrikel aufgenommen werden, hat der Ventrikel Vorrang. Damit ist gewährleistet, daß die Sicherheit gegen schädliche Stimulation aufgrund unerwünschter Überleitungen auch bei nicht eindeutiger Signalerkennung zunächst einmal gegeben ist.

Gemäß einer bevorzugten Ausführung der Erfindung werden alle Umschaltungen (Programmänderungen, Betriebsartänderungen) zu Zeitpunkten vorgenommen, welche den Betriebsablauf nicht stören, das sind solche Zeitpunkte, zu denen keine Stimulation bewirkt werden kann und auch kein Eingangssignal erwartet wird, dessen Signalaufnahme wiederum von den eingestellten Betriebsparametern abhängig sein könnte. Ein derartiger Zeitpunkt ist derjenige auf T$_V$ folgende Zeitbereich, in dem beide Eingangsstufen refraktär sind (vergleiche Fig. 2, aus der die Zeiten des Herzzyklus im Hinblick auf den Betrieb des Schrittmachers in ihrer grundsätzlichen Verteilung ersichtlich sind).

Der mit T$_V$ beginnende Zeitraum ermöglicht dabei die für den Betrieb des Schrittmachers notwendigen Umschaltungen in einer Weise, welche die physiologischen Randbedingungen der Stimulation am wenigsten beeinträchtigen, da Umschaltungen zu dieser Zeit in eine natürliche Ruheperiode des Herzens fallen. Im Zeitpunkt T$_V$ finden auch solche Überprüfungen statt, die regelmäßig durchgeführt werden müssen, wie die Bestimmung des Betriebszustands der Energiequelle (EOL-Block 36).

Der Zeitpunkt T$_V$ wird bei normalem Herzverhalten stets durchlaufen und wäre an sich ausreichend beispielsweise die Übernahme der geänderten Betriebsparameter (entsprechender Pfeil an Speicherblock 4) oder die Änderung der Programmierung aufgrund der von außen her übertragenen entsprechenden Signale zu diesem Zeitpunkt zu bewirken (Pfeil nach Block 13).

## Patentansprüche

1. Herzschrittmacher mit Mitteln zur Signalaufnahme im Atrium und Ventrikel und Mitteln zur Abgabe von Stimulationsimpulsen an das Atrium und den Ventrikel nach Escape-Intervallen, die gegebene zeitliche Abstände zwischen der Signalaufnahme auf eine Herzaktion hin und dem Stimulationszeitpunkt definieren, wobei die Stimulation gegebenenfalls durch eine entsprechende eigene Aktion des Herzens inhibiert wird, sowie mit Schaltmitteln zum starten der Escape-Intervalle, *dadurch gekennzeichnet,* daß die Schaltmittel (213, 213a) zum Starten des Escape-Intervalls für das Atrium entweder einen ersten zeitlichen Abstand zu einer vorangehenden spontanen Aktion bzw. Stimulation des Atriums definieren oder aber einen zweiten zeitlichen Abstand zu einer vorangehenden spontanen Ventrikelaktion, wobei sich das Starten des Escape-Intervalls für das Atriums durch Ventrikelaktionen auf spontane Ventrikelaktionen beschränkt, welche zeitlich zwischen dem Ende der Refraktärzeit für die Signalaufnahme aus dem Ventrikel und dem Ende des letzten Escapeintervalls (T$_A$) für die Atriumaktion erscheinen, so daß die Schaltmittel auf sonstige spontane Ventrikelaktionen hin keine Synchronisation der die Escape-Intervalle steuernden Zeitgeber bewirken.

2. Herzschrittmacher nach Anspruch 1, *dadurch gekennzeichnet,* daß nach spontanen Ventrikelaktionen, welche zeitlich zwischen dem Ende der Refraktärzeit für die Signalaufnahme aus dem Ventrikel und dem Ende des Escape-Intervalls (T$_A$) für Atriumaktion erscheinen, die Atriumrefraktärzeit im wesentlichen 300 ms bis 400 ms, mindestens aber 200 ms beträgt.

3. Herzschrittmacher nach einem der vorangehenden Ansprüche, *dadurch gekennzeichnet daß* Schaltmittel (110) vorgesehen sind, welche bei gleichzeitig auftretenden Signalen im Atrium und Ventrikel die Verarbeitung des Signals aus dem Atrium unterbinden.


## Claims

1. A heart pacemaker with signal pick up means in the atrium and ventricle and means for passing stimulating impulses to the atrium and the ventricle after escape intervals defining predetermined time spacings between the pick up of a signal upon a heart action and the point in time of stimulation, in which the stimulation may in some cases be inhibited by a corresponding natural action of the heart, and having switching means for starting the escape intervals, *characterized in that* the switching means (213, 213a) for starting the escape interval for the atrium either define a first time spacing at a preceding spontaneous action or stimulation of the atrium or a second time spacing at a preceding spontaneous ventricle action, in which start-up of the escape interval for the atrium by ventricle actions is restricted to spontaneous ventricle actions, which arise in time between the end of the refractory time for signal pick up from the ventricle and the end of the last escape interval ($T_A$) for the atrium action, so that the switching means do not cause any synchronisation of the timing generator controlling the escape intervals in response to any spontaneous ventricle actions.

2. A heart pacemaker according to claim 1, *characterized in that* after spontaneous ventricle actions which arise in time between the end of the refractory time for the signal pick up from the ventricle and the end of the escape interval ($T_A$) for atrium action, the atrium refractory time is substantially 300 ms to 400 ms, but at least 200 ms.

3. A heart pacemaker according to any one of the preceding claims, *characterized in that* switching means (110) are provided which suppress processing of the signal from the atrium when signals in the atrium and ventricle occur at the same time.


## Revendications

1. Stimulateur cardiaque comportant des moyens pour recueillir des signaux dans l'oreillette et le ventricule et des moyens pour délivrer des impulsions de stimulation à l'oreillette et au ventricule après des intervalles dits d'échappement (escapeintervals) qui définissent des laps de temps préfixés entre l'enregistrement d'un signal indicateur d'une action du coeur et l'instant de stimulation, avec inhibition éventuelle de la stimulation par une action autonome appropriée du coeur, ainsi que des moyens de couplage pour faire démarrer les intervalles d'échappement *caractérisé en ce* que les moyens de couplage (213, 213a) destinés à faire démarrer l'intervalle d'échappement pour oreillette définissent, soit un premier laps de temps par rapport à une action spontanée ou d'une stimulation précédente de l'oreillette, soit un deuxième laps de temps par rapport à une action spontanée précédente du ventricule, le démarrage de l'intervalle d'échappement pour l'oreillette par des actions du ventricule se limitant à des actions spontanées du ventricule qui apparaissent dans le temps compris entre la fin de la période réfractaire pour l'enregistrement d'un signal provenant du ventricule et la fin du dernier intervalle d'échappement ($T_A$) pour l'action de l'oreillette, de manière que les moyens de couplage ne provoquent pas la synchronisation des moyens d'horloge commandant les intervalles d'échappement à la suite d'autres actions spontanées du ventricule.

2. Stimulateur cardiaque selon la revendication 1, *caractérisé en ce* que, à la suite d'actions spontanées du ventricule apparaissant dans le temps compris entre la fin de la période réfractaire pour l'enregistrement d'un signal provenant du ventricule et la fin de l'intervalle d'échappement ($T_A$) pour l'action de l'oreillette, la période réfractaire de l'oreillette est essentiellement de 300 ms à 400 ms, mais d'au moins 200 ms.

3. Stimulateur cardiaque selon une des revendications précédentes, *caractérisé en ce* qu'il comprend des moyens de couplage (110) qui font cesser le traitement du signal provenant de l'oreillette en cas d'apparition simultanée de signaux dans l'oreillette et dans le ventricule.

Fig. 1

1

Fig. 2